# EUROPÄISCHE PATENTSCHRIFT

(11) **EP 1 927 320 B1**
(45) Veröffentlichungstag und Bekanntmachung des Hinweises auf die Patenterteilung: **23.03.2011**
(21) Anmeldenummer: 07022912.5
(22) Anmeldetag: 27.11.2007
(51) Int. Cl.: A61B 17/28, A61B 17/32

(54) **Medizinisches Instrument**
Medical instrument
Instrument médical

(30) Priorität: 29.11.2006 DE 102006056200
(43) Veröffentlichungstag der Anmeldung: 04.06.2008
(73) Patentinhaber: Karl Storz GmbH & Co. KG, 78532 Tuttlingen (DE)
(72) Erfinder: Teichtmann, Elmar, 78054 Villingen-Schwenningen (DE)
(74) Vertreter: Hofmeister, Frank

(56) Entgegenhaltungen:
- DE-A1- 3 716 764
- DE-A1- 4 444 403
- US-A- 4 662 371

## Beschreibung

Die Erfindung betrifft ein medizinisches Instrument mit einem hohlen Schaft, einem am distalen Ende des Schaftes angeordneten, aus einem starren Maulteil und einem gegenüber dem starren Maulteil verschwenkbaren Maulteil bestehenden Werkzeug sowie einer am proximalen Ende des Schaftes angeordneten Handhabe, wobei das Werkzeug und die Handhabe über eine in dem Schaft (2) gelagerte Schub-/Zugstange miteinander in Wirkverbindung stehen und die Zug-/Schubstange als im Schaft axialverschiebbar gelagertes hohles Schubrohr ausgebildet ist, das über ein im Wesentlichen stabförmiges Betätigungselement mit dem verschwenkbaren Maulteil in Wirkverbindung steht und, wobei die Maulteile des Werkzeugs über angeformte Lagerzapfen verschwenkbar aneinander gelagert sind.

Gattungsgemäße medizinische Instrumente werden in der Praxis häufig als Schneid-, Stanz-, Greif und/oder Haltewerkzeuge eingesetzt. Diesen medizinischen Instrumenten ist gemeinsam, dass das verschwenkbare Maulteil des Werkzeugs möglichst spielfrei und mit ausreichender Kraftübertragung über die Schub-/Zugstange betätigbar ist.

Ein gattungsgemäßes medizinischen Instrument ist aus dem US-Patent 4 662 371 bekannt. Dieses bekannte medizinischen Instrument besteht aus einem hohlen Schaft mit einer proximalen Handhabe sowie einem verschwenkbaren und einem starren Maulteil am distalen Ende des Schaftes. Zum Betätigen des verschwenkbaren Maulteils über einen verschwenkbaren Griffteil der Handhabe ist im hohlen Schaft eine axial verschiebbare Zug-/Schubstange gelagert. Die Zug-/Schubstange ist darüber hinaus als hohles Saugrohr ausgebildet und steht über ein stabförmiges Betätigungselement mit dem verschwenkbaren Maulteil in Wirkverbindung.

Nachteilig bei diesem bekannten medizinischen Instrument ist, dass das starre Maulteil als distales Ende des hohlen Schaftes einstückig mit dem hohlen Schaft ausgebildet, so dass es zur Ausbildung von Instrumenten mit unterschiedlichen Maulteilen immer erforderlich ist, auch den jeweiligen starren Maulteilen angepasste hohle Schäfte vorzuhalten und zu montieren.

Weiterhin ist beispielsweise aus der DE 195 21 257 A1 ein medizinisches Instrument bekannt, bei dem die Lagerung der beiden Maulteile aneinander über am beweglichen Maulteil angeformte Lagerbolzen erfolgt. Die Schub-/Zugstange zum Betätigen der Maulteile erstreckt sich in Längsrichtung durch den hohlen Instrumentenschaft und lässt beidseitig ein sich längs erstreckendes Lumen frei, das als Spülkanal verwendbar ist. Da bei endoskopischen Instrumenten die Außendurchmesser der Instrumentenschäfte in der Regel nur 2 mm oder weniger betragen, bildet dieses zwischen der Schub-/Zugstange und der Schaftinnenseite verbleibende freie Lumen einen Spülkanal mit einem sehr geringen Kanalquerschnitt.

Ein weiteres medizinisches Instrument ist beispielsweise aus dem US-Patent 4 522 206 bekannt. Bei diesem bekannten medizinischen Instrument erfolgt die Lagerung des verschwenkbaren Maulteils am starren Maulteil über zwei in entsprechende Lagerbohrungen in den beiden Maulteilen einzusetzende Achsstummel. Diese Konstruktion ist aus mehrfacher Sicht nachteilig, da einerseits die Verwendung der einzeln in die Lagerbohrungen einzusetzenden Achsstummel bei der Montage kompliziert und zeitintensiv ist und andererseits Instrumente mit sehr kleinem Außendurchmesser mit dieser Ausgestaltung nicht herstellbar sind.

Davon ausgehend liegt der Erfindung die Aufgabe zugrunde, ein medizinisches Instrument der eingangs genannten Art zu schaffen, das bei kleinem Außendurchmesser einfach aufgebaut und leicht zu montieren ist.

Die **Lösung** dieser Aufgabenstellung ist erfindungsgemäß dadurch gekennzeichnet, dass das starre Maulteil des Werkzeugs als mit dem hohlen Schaft dauerhaft verbindbares separates Bauteil ausgebildet ist.

Dadurch, dass das starre Maulteil als vom hohlen Schaft getrenntes separates Bauteil ausgebildet ist, das erst während der Montage dauerhaft mit dem Schaft verbunden wird, beispielsweise durch Verschweißen, ist es möglich, beide das Werkzeug bildende Maulteile als vormontierbare Baueinheit auszubilden und bei Bedarf unterschiedliche Maulteile bei einem an sich unveränderten Instrument einzusetzen, solange die Anschlüsse an das Betätigungselement und den Schaft bestehen bleiben.

Die Ausgestaltungsform mit dem hohlen Schubrohr ist insbesondere dann vorteilhaft, wenn die Benutzung des hohlen Instrumentenschaftes als Saug- und/oder Spülkanal ermöglicht werden soll.

Um eine besonders gute Kraftübertragung von der Zug-/Schubstange auf das verschwenkbare Maulteil zu gewährleisten, ist das stabförmige Betätigungselement mit einem Ende drehbar am verschwenkbaren Maulteil gelagert und mit dem anderen Ende mit dem Schubrohr verbunden. Vorteilhafterweise ist das Betätigungselement federelastisch ausgebildet bzw. federelastisch gelagert, um bei der rein axialen Verschiebung des Schubrohres dem bogenförmigen Verlauf des Anlenkpunktes des Betätigungselements am verschwenkbaren Maulteil folgen zu können, wobei das stabförmige Betätigungselement und das verschwenkbare Maulteil erfindungsgemäß auf einem gemeinsamen Lagerstift gelagert sind.

Durch die direkte Anformung der Lagerzapfen an die Maulteile des Werkzeugs wird die Anzahl der bei der Montage miteinander zu verbindenden Bauteile reduziert und dadurch die Montage vereinfacht. Darüber hinaus ermöglicht die erfindungsgemäße Ausgestaltung auch die Herstellung von Instrumenten mit einem kleinen Außendurchmesser von beispielsweise 2 mm und weniger, da die angeformten Lagerzapfen nicht noch vernietet oder verschweißt werden müssen.

Gemäß einer bevorzugten Ausführungsform der Erfindung wird vorgeschlagen, dass an einem Maulteil zwei Lagerzapfen angeordnet sind, die in entsprechende Lageraufnahmen des anderen Maulteils einsetzbar sind. Durch die Ausbildung der beiden Lagerzapfen an nur einem der beiden Maulteile wird die Montage vereinfacht.

Gemäß einer ersten praktischen Ausführungsform der Erfindung wird vorgeschlagen, dass die Lagerzapfen am verschwenkbaren Maulteil angeordnet sind und die Lageraufnahmen als Bohrungen im starren Maulteil ausgebildet sind.

Mit einer zweiten praktischen Ausführungsform der Erfindung wird vorgeschlagen, dass die Lagerzapfen am verschwenkbaren Maulteil angeordnet sind und die Lageraufnahmen als im starren Maulteil und im Schaft ausgeformte Lagersegmente ausgebildet sind. Diese Ausgestaltung der Lageraufnahmen als halb im starren Maulteil und halb im Schaftrohr ausgebildet stellt eine für die Montage besonders einfache und schnelle Bauweise dar.

Schließlich wird mit der Erfindung vorgeschlagen, dass das verschwenkbare Maulteil und die Schub-/Zugstange als vormontierbare Baueinheit ausgebildet sind. Zusätzlich zu der Reduzierung der Bauteilzahl durch das erfindungsgemäße direkte Anformen der Lagerzapfen an die Maulteile kann die Montage weiterhin dadurch vereinfacht werden, dass einzelne Bauteile, wie beispielsweise das verschwenkbare Maulteil und die Schub-/Zugstange, als Bauteilgruppen vormontierbar sind, die dann bei Bedarf nur noch mit den restlichen Bauteilen zu verbinden sind.

Weitere Merkmale und Vorteile der Erfindung ergeben sich anhand der zugehörigen Zeichnung, in der zwei Ausführungsbeispiele eines erfindungsgemäßen medizinischen Instruments nur beispielhaft dargestellt sind, ohne die Erfindung auf diese Ausführungsbeispiele zu beschränken. In der Zeichnung zeigt:
- Fig. 1: eine schematische Seitenansicht eines erfindungsgemäßen medizinischen Instruments;
- Fig. 2: eine perspektivische Ansicht des das distale Endes des medizinischen Instruments darstellenden Details II gemäß Fig. 1, eine erste erfindungsgemäße Ausführungsform darstellend;
- Fig. 3: eine teilweise geschnittene Seitenansicht der Darstellung gemäß Fig. 2;
- Fig. 4: eine perspektivische Zusammenschau der einzelnen Bauteile des distalen Endes gemäß Fig. 2 und 3;
- Fig. 5: eine Seitenansicht des das distale Endes des medizinischen Instruments darstellenden Details V gemäß Fig. 1, eine zweite erfindungsgemäße Ausführungsform darstellend und
- Fig. 6: eine perspektivische Zusammenschau der einzelnen Bauteile des distalen Endes gemäß Fig. 5.

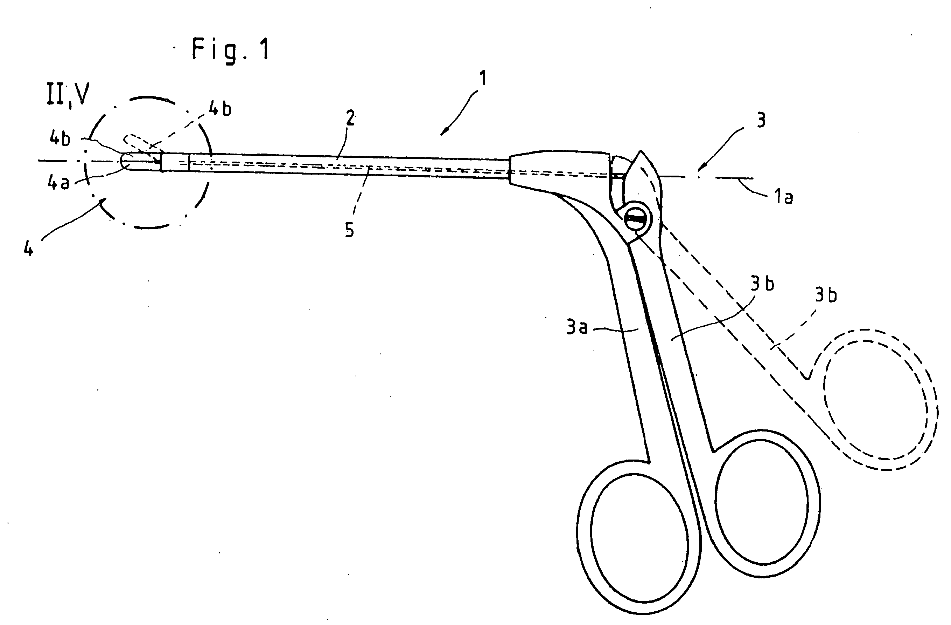

Die Abbildung Fig. 1 zeigt die Seitenansicht eines medizinischen Instruments, dessen Kraftübertragungsmechanismus vielseitig verwendet werden kann, wie beispielsweise für Stanzen, Scheren, Nadelhalter, Fassinstrumente und dergleichen.

Das nur exemplarisch dargestellte medizinische Instrument 1 besteht im wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen ende des Schaftes 2 ist ein Werkzeug 4 angeordnet, das von einem starren Maulteil 4a und einem gegenüber dem starren Maulteil 4a verwschwenkbaren Maulteil 4b besteht.

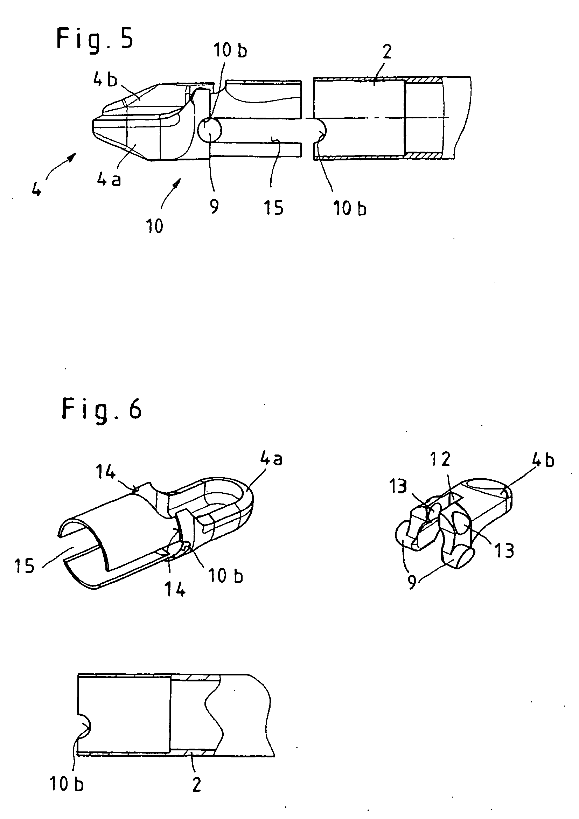

Wie aus Fig. 1 ersichtlich, stehen das verschwenkbare Maulteil 4b des Werkzeugs 4 und der verschwenkbare Griffteil 3b der Handhabe 3 über eine in dem hohlen Schaft 2 axial verschiebbar gelagerte Zug-/Schubstange 5 derart in Wirkverbindung miteinander, dass durch das Verstellen des Griffteils 3b der Handhabe 3 das Maulteil 4b des Werkzeugs 4 von der geschlossenen Stellung (durchgezogene Darstellung gemäß Fig. 1 sowie Fig. 5) in die geöffnete Stellung (gestrichelte Darstellung gemäß Fig. 1 sowie Fig. 2 und 3) bzw. umgekehrt überführbar ist. Die jeweils zugehörige Stellung des verschwenkbaren Griffteils 3b der Handhabe 3 ist in der Abbildung Fig. 1 ebenfalls durchgezogen (für die geschlossene Stellung) und gestrichelt (für die geöffnete Stellung) dargestellt.

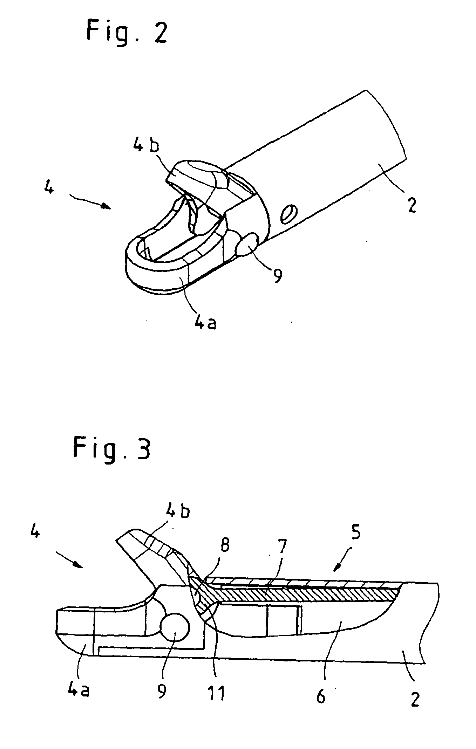

Wie aus Fig. 3 ersichtlich, besteht die Zug-/Schubstange 5 bei dem dargestellten medizinischen Instrument 1 aus einem axialverschiebbar im hohlen Schaft 2 gelagerten hohlen Schubrohr 6 und einem im Wesentlichen stabförmigen Betätigungselement 7, welches in einem Schlitz 6a des Schubrohres 6 festlegbar die eigentliche Verbindung zwischen der Zug-/Schubstange 5 und dem verschwenkbaren Maulteil 4b bildet. Hierzu ist das stabförmige Betätigungselement 7 mit einem Ende über einen Lagerstift 8 drehbar am verschwenkbaren Maulteil 4b des Werkzeugs 4 gelagert ist und mit dem anderen Ende mit dem Schubrohr 6 verbunden. Die Ausbildung der Zug-/Schubstange 5 mit einem hohlen Schubrohr 6 wird insbesondere bei medizinischen Instrumenten 1, wie beispielsweise Saugstanzen, verwendet, deren hohler Schaft 2 als Saug- und/oder Spülkanal dient.

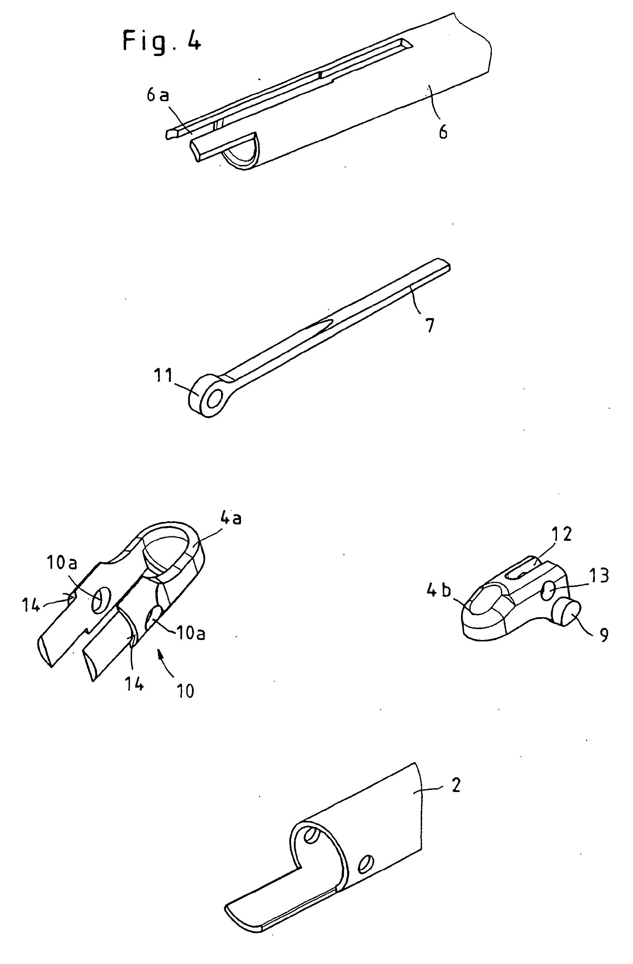

Der Aufbau der Maulteile 4a und 4b des Werkzeugs 4 ergibt sich insbesondere aus den Abbildungen Fig. 4 (für die erste Ausführungsform) und Fig. 6 (für die zweite Ausführungsform). Wie aus den beiden Abbildungen Fig. 4 und Fig. 6 in Zusammenschau mit den zugehörigen Abbildungen Fig. 3 und Fig. 5 ersichtlich, sind die Maulteile 4a, 4b bei den dargestellten Ausführungsformen so ausgebildet, dass an den verschwenkbaren Maulteilen 4b seitlich nach außen vorspringenden Lagerzapfen 9 angeformt sind, über die die verschwenkbaren Maulteile 4b an den starren Maulteilen 4a lagerbar sind. Zur Aufnahme der Lagerzapfen 9 des jeweiligen verschwenkbaren Maulteils 4b weist das zugehörige starre Maulteil 4a den Lagerzapfen 9 entsprechende Lageraufnahmen 10 auf.

Alternativ zu den dargestellten Ausführungsformen mit ausschließlich am verschwenkbaren Maulteil 4b angeformten Lagerzapfen 9 ist es selbstverständlich auch möglich, die Lagerzapfen 9 ausschließlich am starren Maulteil 4a oder aber jeweils einen Lagerzapfen 9 am starren Maulteil 4a und einen Lagerzapfen am verschwenkbaren Maulteil 4b auszubilden, wobei dann am jeweils anderen Maulteil 4a oder 4b die entsprechenden Lageraufnahmen 10 zur lagernden Aufnahme der Lagerzapfen 9 ausgebildet sind.

Das Ausbilden der Lagerstellen der Maulteile 4a und 4b als direkt am Maulteil 4b angeformte Lagerzapfen 9 hat den Vorteil, dass die Lagerstelle ganz an den Rand des Bauteils verschoben werden kann und keine Wandung oder Materialstärke benötigt, um die aus dem Stand der Technik bekannten Bohrungen zur Aufnahme eines Lagerstiftes auszubilden. Diese Verlagerung der Lagerstelle nach außen an den Rand des Bauteils bringt Vorteile für die Hebellänge und somit für die Kraftübertragung, da so ein größerer Abstand zwischen der Lagerstelle des verschwenkbaren Maulteils 4b und dem Angriffspunkt des Betätigungselements 7 der Zug-/Schubstange 5 am verschwenkbaren Maulteil 4b realisierbar ist.

Die beiden in den Abbildungen Fig. 2 bis 4 sowie Fig. 5 und 6 dargestellten Ausführungsformen unterscheiden sich durch die Ausbildung der zur Aufnahme der Lagerzapfen 9 des verschwenkbaren Maulteils 4b dienenden Lageraufnahmen 10 voneinander.

Bei der ersten Ausführungsform gemäß Fig. 2 bis 4 ist die Lageraufnahme 10 als Lagerbohrung 10a im starren Maulteil 4a ausgebildet, wobei in den Seitenwänden des starren Maulteils 4a jeweils eine Lagerbohrung 10a ausgebildet ist, die im fertig montierten Zustand des Werkzeugs 4 die Lagerzapfen 9 des verschwenkbaren Maulteils 4b vollumfänglich umschließen.

Dahingegen bestehen die Lageraufnahmen 10 bei der in den Abbildungen Fig. 5 und 6 dargestellten zweiten Ausführungsform aus im starren Maulteil 4a sowie im Schaft 2 ausgebildeten Lagersegmenten 10b, die erst im fertig montierten Zustand von Werkzeug 4 und Schaft 2 die Lagerzapfen 9 vollumfänglich umschließende Lageraufnahmen 10 bilden.

Der Zusammenbau der in den Abbildungen Fig. 2 bis 6 dargestellten distalen Bereiche des medizinischen Instruments 1 geschieht wie folgt:

In einem ersten Arbeitsschritt wird das Betätigungselement 7 zur Komplettierung der Zug-/Schubstange 5 in den Schlitz 6a des Schubrohres 6 eingesetzt und mit dem Schubrohr 6 verschweißt. Zur Verbindung des Betätigungselements 7 mit dem verschwenkbaren Maulteil 4b ist im distalen Ende des Betätigungselements 7 eine Öse 11 zur Aufnahme des Lagerstiftes 8 ausgebildet, die so in einen im proximalen Ende des verschwenkbaren Maulteil 4b ausgebildeten Einschnitt 12 einsetzbar ist, dass die Öse 11 mit im verschwenkbaren Maulteil 4b ausgebildeten zwei Lagerbohrungen 13 fluchtet. In diesem Zustand wird nun das Betätigungselement 7 über den Lagerstift 8 dauerhaft mit dem verschwenkbaren Maulteil 4b verbunden, so dass die Zug-/Schubstange 5 mitsamt dem verschwenkbaren Maulteil 4b als vorfertigbare Baueinheit ausgebildet sind.

Da sich der Lagerstift 8 und das auf dem Lagerstift 8 gelagerte Betätigungselement 7 beim Verschwenken des Maulteils 4b auf einem Kreisbogen um die Lagerstelle des verschwenkbaren Maulteils 4b am starren Maulteil 4a bewegt, ist es vorteilhaft, das Betätigungselement 7 möglichst weit proximal im Schlitz 6a mit dem Schubrohr 6 zu verschweißen, um das Betätigungselement 7 federelastisch auszubilden.

Um eine Überlastung des Lagerstifts 8 zu verhindern, können im Inneren des Schaftes oder des Schubrohres 6 Anschlagflächen ausgebildet sein, gegen die das verschwenkbare Maulteil 4b in der geschlossenen Stellung anläuft. Diese Anschlagflächen verhindern ein zu tiefes Eintauchen des verschwenkbaren Maulteils 4b in das starre Maulteil 4a beim Schließen des Werkzeugs 4.

Dieser vorgenannte Arbeitsschritt mit der Ausbildung der vorgefertigten Baueinheit aus Zug-/Schubstange 5 und verschwenkbarem Maulteil 4b ist für beide dargestellten Ausführungsformen identisch.

Zur Komplettierung des Werkzeugs 4 müssen nachfolgend das verschwenkbare Maulteil 4b und das starre Maulteil 4a miteinander verbunden werden. Aufgrund der unterschiedlich ausgebildeten Lageraufnahmen 10 bei den beiden dargestellten Ausführungsformen eines medizinischen Instruments 1 unterscheiden sich die nachfolgenden Arbeitsschritte zur Montage der beiden medizinischen Instrumente 1 voneinander.

Bei der in Fig. 2 bis 4 dargestellten ersten Ausführungsform wird zunächst das gabelförmig ausgebildete proximale Ende des starren Maulteils 4a durch Auseinanderbiegen der Seitenwände aufgespreizt und nachfolgend das verschwenkbare Maulteil 4b soweit in das aufgespreizte proximale Ende des starren Maulteils 4a eingesetzt, bis die angeformten Lagerzapfen 9 mit den Lagerbohrungen 10a fluchten. Sobald die Lagerzapfen 9 in die Lagerbohrungen 10a eintreten, springt das aufgespreizte proximale Ende des starren Maulteils 4a wieder in seine Ausgangslage zurück und fixiert die beiden Maulteile 4a und 4b aneinander.

Alternativ ist es jedoch auch möglich, das durch den Einschnitt 12 ebenfalls gabelförmig ausgebildete proximale Ende des verschwenkbaren Maulteils 4b zusammenzudrücken und nachfolgend in das gabelförmige proximale Ende des starren Maulteils 4a einzusetzen, bis die angeformten Lagerzapfen 9 mit den Lagerbohrungen 10a fluchten. Sobald die Lagerzapfen 9 mit den Lagerbohrungen 10a fluchten, springt das zusammengedrückte proximale Ende des verschwenkbaren Maulteils 4b wieder in seine Ausgangslage zurück und fixiert die beiden Maulteile 4a und 4b aneinander.

Anschließend wird der hohle Schaft 2 vom proximalen Ende über das Schubrohr 6 geschoben, bis das distale Ende des Schaftes 2 an Anschlagflächen 14 des starren Maulteils 4a anliegt und dauerhaft, beispielsweise durch Schweißen, mit dem starren Maulteil 4a verbunden.

Bei der in Fig. 5 und 6 dargestellten zweiten Ausführungsform sind in das proximale Ende des starren Maulteils 4a zwei einander gegenüberliegende Langlöcher 15 zur Aufnahme der Lagerzapfen des verschwenkbaren Maulteils 4b ausgebildet. Die Lagersegmente 10b bilden die distalen Enden der Länglöcher 15. In einem ersten Schritt zur Komplettierung des Werkzeugs 4 werden die angeformten Lagerzapfen 9 des verschwenkbaren Maulteils 4b in die Langlöcher 15 eingesetzt und das verschwenkbare Maulteil 4b soweit in distaler Richtung geschoben, bis die Lagerzapfen 9 die Lagersegmente 10b und somit das distale Ende der Langlöcher 15 erreicht haben.

Anschließend wird der hohle Schaft 2 vom Ende über das Schubrohr 6 geschoben, bis das distale Ende des Schaftes 2 an Anschlagflächen 14 des starren Maulteils 4a anliegt. Zur Komplettierung der Lageraufnahme 10 sind im distalen Ende des Schaftes 2 halbkreisförmige Ausnehmungen als Lagersegmente 10b ausgebildet, die im auf das starre Maulteil 4a aufgeschobenen Zustand mit den Lagersegmenten 10b des starren Maulteils 4 die Lagerzapfen 9 vollumfänglich umschließende Lageraufnahmen 10 bilden.

Wie zuvor beschrieben ausgebildete medizinische Instrumente 1 zeichnen sich dadurch aus, dass sie einfach aufgebaut und leicht zu montieren sind und darüber hinaus die Herstellung medizinischer Instrumente mit geringem Außendurchmesser ermöglichen.

### Bezugszeichenliste

- 1: medizinisches Instrument
- 2: Schaft
- 3: Handhabe
- 3a: starrer Griffteil
- 3b: verschwenkbarer Griffteil
- 4: Werkzeug
- 4a: starres Maulteil
- 4b: verschwenkbares Maulteil
- 5: Zug-/Schubstange
- 6: Schubrohr
- 6a: Schlitz
- 7: Betätigungselement
- 8: Lagerstift
- 9: Lagerzapfen
- 10: Lageraufnahme
- 10a: Lagerbohrung
- 10b: Lagersegment
- 11: Öse
- 12: Einschnitt
- 13: Lagerbohrung
- 14: Anschlagfläche
- 15: Langloch

## Patentansprüche

1. Medizinisches Instrument mit einem hohlen Schaft (2), einem am distalen Ende des Schaftes (2) angeordneten, aus einem starren Maulteil (4a) und einem gegenüber dem starren Maulteil (4a) verschwenkbaren Maulteil (4b) bestehenden Werkzeug (4) sowie einer am proximalen Ende des Schaftes (2) angeordneten Handhabe (3), wobei das Werkzeug (4) und die Handhabe (3) über eine in dem Schaft (2) gelagerte Schub-/Zugstange (5) miteinander in Wirkverbindung stehen und die Zug-/Schubstange (5) als im Schaft (2) axialverschiebbar gelagertes hohles Schubrohr (6) ausgebildet ist, das über ein im Wesentlichen stabförmiges Betätigungselement (7) mit dem verschwenkbaren Maulteil (4b) in Wirkverbindung steht und, wobei die Maulteile (4a, 4b) des Werkzeugs (4) über angeformte Lagerzapfen (9) verschwenkbar aneinander gelagert sind,
**dadurch gekennzeichnet,**
**dass** das starre Maulteil (4a) des Werkzeugs (4) als mit dem hohlen Schaft (2) dauerhaft verbindbares separates Bauteil ausgebildet ist.

2. Medizinisches Instrument nach Anspruch 1, **dadurch gekennzeichnet, dass** die Maulteile (4a und 4b) des Werkzeugs (4) vor dem Verbinden mit dem Schaft (2) aneinander festlegbar sind.

3. Medizinisches Instrument nach Anspruch 1, oder 2 **dadurch gekennzeichnet, dass** das stabförmige Betätigungselement (7) mit einem Ende drehbar am verschwenkbaren Maulteil (4b) gelagert ist und mit dem anderen Ende mit dem Schubrohr (6) verbunden ist.

4. Medizinisches Instrument nach einem der Ansprüche 1 bis 3, **dadurch gekennzeichnet, dass** das stabförmige Betätigungselement (7) federelastisch ausgebildet ist.

5. Medizinisches Instrument nach Anspruch 3 oder 4, **dadurch gekennzeichnet, dass** das stabförmige Betätigungselement (7) und das verschwenkbare Maulteil (4b) auf einem gemeinsamen Lagerstift (8) gelagert sind.

6. Medizinisches Instrument nach einem der Ansprüche 1 bis 5, **dadurch gekennzeichnet, dass** die Lagerzapfen (9) der Maulteile (4a und/oder 4b) in entsprechende Lageraufnahmen (10) der jeweils anderen Maulteile (4b und/oder 4a) einsetzbar sind.

7. Medizinisches Instrument nach Anspruch 6, **dadurch gekennzeichnet, dass** an einem Maulteil (4a oder 4b) zwei Lagerzapfen (9) angeordnet sind, die in entsprechende Lageraufnahmen (10) des anderen Maulteils (4b oder 4a) einsetzbar sind.

8. Medizinisches Instrument nach Anspruch 7, **dadurch gekennzeichnet, dass** die Lagerzapfen (9) am verschwenkbaren Maulteil (4b) angeordnet sind und die Lageraufnahmen (10) als Lagerbohrungen (10a) im starren Maulteil (4a) ausgebildet sind.

9. Medizinisches Instrument nach Anspruch 6, **dadurch gekennzeichnet, dass** die Lagerzapfen (9) am verschwenkbaren Maulteil (4b) angeordnet sind und die Lageraufnahmen (10) als im starren Maulteil (4a) und im Schaft (2) ausgeformte Lagersegmente (10b) ausgebildet sind.

10. Medizinisches Instrument nach einem der Ansprüche 1 bis 9, **dadurch gekennzeichnet, dass** das verschwenkbare Maulteil (4b) und die Schub-/Zugstange (5) als vormontierbare Baueinheit ausgebildet sind.

## Claims

1. Medical instrument with a hollow shaft (2), a tool (4) arranged on the distal end of the shaft (2) and composed of a rigid jaw member (4a) and a jaw member (4b) that can be rotated with respect to the rigid jaw member (4a) and a handle (3) arranged on the proximal end of the shaft (2), wherein the tool (4) and the handle (3) are in active engagement with one another via a push/pull rod (5) supported in the shaft (2) and the push/pull rod (5) is embodied as a hollow slide tube (6) supported in the shaft (2) in an axially slidable manner, which slide tube is in active engagement with the rotatable jaw member (4b) via an essentially rod-shaped actuating element (7), and wherein the jaw members (4a, 4b) of the tool (4) are supported on one another in a rotatable manner via integrally shaped journal pins (9), **characterized in that** the rigid jaw member (4a) of the tool (4) is embodied as a separate component that can be permanently connected to the hollow shaft (2).

2. Medical instrument according to claim 1, **characterized in that** the jaw members (4a and 4b) of the tool (4) can be fixed to one another before connection to the shaft (2).

3. Medical instrument according to claim 1 or 2, **characterized in that** the rod-shaped actuating element (7) is rotatably mounted with one end on the rotatable jaw member (4b) and with the other end is connected to the slide tube (6).

4. Medical instrument according to one of claims 1 through 3, **characterized in that** the rod-shaped actuating element (7) is embodied in a spring elastic manner.

5. Medical instrument according to claim 3 or 4, **characterized in that** the rod-shaped actuating element (7) and the rotatable jaw member (4b) are supported on a common bearing pin (8).

6. Medical instrument according to one of claims 1 through 5, **characterized in that** the journal pins (9) of the jaw members (4a and/or 4b) can be inserted into corresponding bearing seats (10) of the respectively other jaw members (4b and/or 4a).

7. Medical instrument according to claim 6, **characterized in that** two journal pins (9) are arranged on one jaw member (4a or 4b), which journal pins can be inserted into corresponding bearing seats (10) of the other jaw member (4b or 4a).

8. Medical instrument according to claim 7, **characterized in that** the journal pins (9) are arranged on the rotatable jaw member (4b) and the bearing seats (10) are embodied as bearing bores (10a) in the rigid jaw member (4a).

9. Medical instrument according to claim 6, **characterized in that** the journal pins (9) are arranged on the rotatable jaw member (4b) and the bearing seats (10) are embodied as bearing segments (10b) shaped out of the rigid jaw member (4a) and the shaft (2).

10. Medical instrument according to one of claims 1 through 9, **characterized in that** the rotatable jaw member (4b) and the push/pull rod (5) are embodied as an assembly that can be preassembled.

## Revendications

1. Instrument médical comprenant un corps allongé creux (2), un outil (4) agencé à l'extrémité distale du corps allongé (2) et constitué d'une pièce de mâchoire (4a) fixe et d'une pièce de mâchoire (4b) pouvant pivoter par rapport à la pièce de mâchoire fixe (4a), ainsi qu'une poignée de manoeuvre (3) agencée à l'extrémité proximale du corps allongé (2), l'outil (4) et la poignée de manoeuvre (3) étant reliés de manière interactive par l'intermédiaire d'une tige de traction/poussée (5) montée dans le corps allongé (2), et la tige de traction/poussée (5) étant réalisée sous la forme d'un tube de poussée (6) creux, qui est monté axialement coulissant dans le corps allongé (2) et est en liaison interactive, par l'intermédiaire d'un élément d'actionnement (7) sensiblement en forme de barre, avec la pièce de mâchoire (4b) pouvant pivoter, et les pièces de mâchoire (4a, 4b) de l'outil (4) étant montées de manière pivotante l'une sur l'autre par l'intermédiaire de tourillons de palier (9) qui y sont formés ou façonnés,
**caractérisé en ce que** la pièce de mâchoire (4a) fixe de l'outil (4) est réalisée en tant que pièce de construction séparée ou distincte pouvant être reliée de manière permanente au corps allongé (2) creux.

2. Instrument médical selon la revendication 1, **caractérisé en ce que** les pièces de mâchoire (4a et 4b) de l'outil (4) peuvent être fixées l'une à l'autre avant la liaison avec le corps allongé (2).

3. Instrument médical selon la revendication 1 ou la revendication 2, **caractérisé en ce que** l'élément d'actionnement (7) en forme de barre est monté rotatif, avec une extrémité, sur la pièce de mâchoire (4b) pouvant pivoter, et est relié, par l'autre extrémité, au tube de poussée (6).

4. Instrument médical selon l'une des revendications 1 à 3, **caractérisé en ce que** l'élément d'actionnement (7) en forme de barre est d'une configuration à élasticité de ressort.

5. Instrument médical selon la revendication 3 ou la revendication 4, **caractérisé en ce que** l'élément d'actionnement (7) en forme de barre et la pièce de mâchoire (4b) pouvant pivoter sont montés sur une broche de palier (8) commune.

6. Instrument médical selon l'une des revendications 1 à 5, **caractérisé en ce que** les tourillons de palier (9) des pièces de mâchoire (4a et/ou 4b) peuvent être insérés dans des logements de palier (10) correspondants des autres pièces de mâchoire respectives (4b et/ou 4a).

7. Instrument médical selon la revendication 6, **caractérisé en ce que** sur une pièce de mâchoire (4a ou 4b) sont agencés deux tourillons de palier (9), qui peuvent être insérés dans des logements de palier (10) correspondants de l'autre pièce de mâchoire (4b ou 4a).

8. Instrument médical selon la revendication 7, **caractérisé en ce que** les tourillons de palier (9) sont agencés sur la pièce de mâchoire (4b) pouvant pivoter, et les logements de palier (10) sont réalisés en tant qu'alésages de palier (10a) dans la pièce de mâchoire (4a) fixe.

9. Instrument médical selon la revendication 6, **caractérisé en ce que** les tourillons de palier (9) sont agencés sur la pièce de mâchoire (4b) pouvant pivoter, et les logements de palier (10) sont réalisés en tant que segments de palier (10b) formés ou façonnés dans la pièce de mâchoire (4a) fixe et dans le corps allongé (2).

10. Instrument médical selon l'une des revendications 1 à 9, **caractérisé en ce que** la pièce de mâchoire (4b) pouvant pivoter et la tige de traction/poussée (5) sont réalisées en tant que module de construction pouvant être monté préalablement.
